# EUROPEAN PATENT APPLICATION

(11) **EP 3 501 570 A1**
(43) Date of publication of application: **26.06.2019**
(21) Application number: 17210432.5
(22) Date of filing: 22.12.2017
(51) Int. Cl.: A61M 5/142, A61M 5/315

(54) **DRUG DELIVERY DEVICE WITH CONTAINER HAVING A PLUNGER**

(71) Applicant: Sensile Medical AG, 4600 Olten (CH)
(72) Inventor: RICKENBACHER, TIMON, 4125 Riehen (CH)
(74) Representative: reuteler & cie SA

(57) **Abstract**

Drug delivery device comprising a suction pump (2) and a liquid drug container (4), the container comprising a tubular container wall (8) extending from an outlet (10) fluidly connected to the suction pump (2) and a rear open end within which a plunger (6) is inserted, the plunger comprising an elastic sealing plug (12) with sealing ring lips (22) compressed against an inner surface of the tubular container wall hermetically sealing a liquid drug within the container. The plunger further comprises a rigid support body (14), the rigid support body comprising a plug insert portion (24) comprising a head portion (28) mounted within an inner cavity (20) of the elastic sealing plug (12) and a trailing end portion (26) extending rearwardly from the head portion (28) and comprising at least one guide rim (32) having a radial guide surface (44) slidably engaging the container wall. The at least one guide rim (32) extends up to a distance (*L*) from a front wall portion (16) of the elastic sealing plug (12) of at least 80% of an overall outer diameter (D) of the plunger.

## Description

### TECHNICAL FIELD

The present invention relates to a drug delivery device having a container with a plunger for containing a liquid drug.

### DESCRIPTION OF RELATED ART

Conventional drug delivery devices with liquid drug containers exist in many different configurations. One of the common configurations comprises a cartridge filled with a liquid drug having on one end an outlet for the liquid drug and at the other end a slidable sealing plunger driven by a piston rod coupled to a pump motor. The plunger is typically of an elastomeric polymer material that has annular sealing lips that engage elastically against the container inner wall. Due to the stick-slip effect of frictional engagement between the sealing lips and container wall, the pump motor requires considerable force to initiate movement of the plunger in comparison to the force required when the plunger is sliding during drug delivery. Also, the piston rod installed behind the plunger and the travel of the piston from the cartridge-full to cartridge-empty position requires a long drug delivery device relative to the volume of liquid contained in the drug cartridge.

The use of drug delivery devices with slidable sealing plungers is however convenient for various reasons because of the rigid container wall. For instance, a rigid transparent cartridge wall allows the volume of liquid in the container to be easily ascertained. Also, the container material may be made of glass or other rigid material that has a low gas permeability.

Containers without plungers are also known, for instance supple containers that collapse as they are emptied. In such systems, liquid can easily been drawn up by a pump system that presses an actuator against the supple container, or by a pump that draws liquid out of the container by suction. The use of supple containers is however often not desired due various drawbacks that may include the material properties of supple containers, an inability to ascertain visually the fill level, gas permeability, poor protection against perforation, and difficult assembly in a drug delivery device.

### SUMMARY OF THE INVENTION

In view of the foregoing, it is an object of the invention to provide a drug delivery device having a container with a plunger for containing a liquid drug, which is compact and economical to produce, yet allows reliable and accurate delivery of a liquid drug.

Disclosed herein is a drug delivery device comprising a suction pump and a liquid drug container, the container comprising a tubular container wall extending from an outlet fluidly connected to the suction pump and a rear open end within which a plunger is inserted, the plunger comprising an elastic sealing plug with sealing ring lips compressed against an inner surface of the tubular container wall hermetically sealing a liquid drug within the cartridge. The plunger further comprises a rigid support body, the rigid support body comprising a plug insert portion comprising a head portion mounted within an inner cavity of the elastic sealing plug and a trailing end portion extending rearwardly from the head portion and comprising at least one guide rim having a radial guide surface slidably engaging the container wall. The at least one guide rim extends up to a distance *L* from a front wall portion of the elastic sealing plug of at least 80% of an overall outer diameter *D* of the plunger.

In an advantageous embodiment, the trailing end portion of the rigid support body comprises at least two guide rims separated by an annular recess.

In an advantageous embodiment, said at least one guide rim is arranged at an end of the trailing end portion and at least the second guide rim is positioned adjacent and in contact with a rear end of the elastic sealing plug.

In an advantageous embodiment, the head portion of the rigid support body comprises an outer diameter *Ds* that is at least 60% of an outer diameter *D* of the plunger.

In an advantageous embodiment, the plug insert portion comprises an annular recess interconnecting the head portion to the trailing end portion, and the elastic sealing plug comprising an inner annular ring portion complementary to the annular recess and mounted in the annular recess of the plug insert portion for fixedly coupling the elastic sealing plug to the rigid support body.

In an advantageous embodiment, the elastic sealing plug comprises at least two sealing ring lips, at least one of the sealing ring lips being positioned around and axially aligned with the head portion of the rigid support body.

In an advantageous embodiment, the elastic sealing ring comprises at least three sealing ring lips, at least two of the sealing ring lips axially aligned with and extending around the plug insert portion.

In an advantageous embodiment, the elastic sealing plug comprises at least one sealing ring lip extending around a front wall portion of the elastic sealing plug axially offset from an end of the head portion of the rigid support body.

In an advantageous embodiment, a front wall portion of the elastic sealing plug has a thickness *Lt* in a range of 10% to 30% of an outer diameter *D* of the plunger.

In an advantageous embodiment, the rigid support body trailing end length (*Ls*) is at least 30% of the overall plunger length *L.*

In an advantageous embodiment, the rigid support body trailing end length *Ls* is in a range of 40-60% of the overall plunger length *L.*

Further objects and advantageous features of the invention will be apparent from the claims, from the detailed description, and annexed drawings, in which:

### BRIEF DESCRIPTION OF THE DRAWINGS

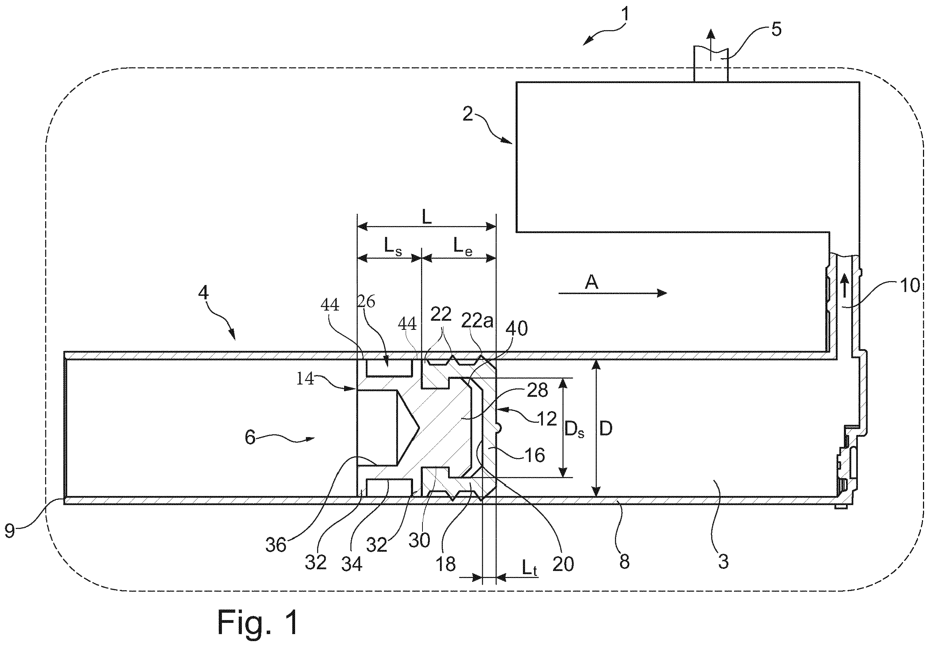

Figure 1 is a cross-sectional schematic view of part of a drug delivery device having a container with a plunger for containing a liquid drug, according to an embodiment of the invention.

### DETAILED DESCRIPTION OF EXEMPLARY EMBODIMENTS

Referring to the figure, a portion of a drug delivery device 1 is schematically represented (without housing and various components) including a container 4 containing a liquid drug 3 to be administered, and a suction pump 2 fluidly connected to an outlet 10 of the container 4 for administering the liquid drug 3 to a patient or for pumping the liquid drug out of the container. The container outlet 10 thus leads into an inlet of the pump. The pump outlet 5 may be connected to a catheter or transcutaneous needle or other means to deliver the liquid drug to a patient. The liquid container 4 may be integrally formed with a housing or other parts of the drug delivery device, or may be in the form of an independent cartridge that is installed in the housing of the drug delivery device. An independent cartridge container may for instance comprise a septum at the outlet end that is perforated by a needle in the device, the needle forming the container outlet fluidly connected to an inlet of the suction pump 2. The container and suction pump 1 may be integrated in a disposable (non-reusable) unit that may be coupled to a reusable unit comprising a pump drive, power source and control electronics. The drug delivery device may be provided in different forms for different uses, an example being in the form of a patch unit for mounting against a patient's skin.

The container 4 comprises a cylindrical tubular container wall 8 extending from a rear end 9 to an outlet 10. A slidable plunger 6 is mounted within the container 4 and sealingly closes a rear end of the container opposite the outlet 10. As liquid contained within the container is drawn out by the suction pump 2, the plunger 6 slidably displaces towards the outlet 10.

The pump 2 is a suction pump that draws liquid out of the container by applying negative pressure on the outlet. The absence of a piston rod mechanism to push the plunger advantageously reduces the size of the drug delivery device and reduces the number of components.

Various suction pumps may be used within the scope of the invention, however a particularly advantageous pump that draws liquid by applying negative pressure and that is useful in medical applications due to its reliability, accuracy and compactness, is described in EP1803934 and in EP1677859. One of the advantageous of the aforementioned pump is that there is no direct communication between the inlet and outlet of the pump because the inlet and outlet valves of the pump can never be open at the same time. This characteristic ensures safe operation of the pump in particular for medical applications whereby a drug may only be administered when the pump is being operated, but liquid through-flow is automatically prevented when the pump stops operating, at any position of the pump.

The plunger 6 comprises an elastic sealing plug 12 and a rigid support body 14. The elastic sealing plug 12 comprises a front wall portion 16 and a tube portion 18 surrounding an inner cavity 20. At least two sealing ring lips 22 on the outer diameter of the tube portion 18 elastically engage in compression an inner surface of the container wall to the ensure hermetic sealing between the sealing plug 12 and the container wall to close the rear end of the container. The rigid support body 14 is coupled to the elastic sealing plug 12 and extends from the trailing end of the elastic sealing plug.

The rigid support body 14 comprises a plug insert portion 24 mounted in the inner cavity 20 of the elastic sealing plug 12 and a trailing end portion 26.

The plunger has a length *L* and an outer diameter *D.* The axial plunger length *L* is comprised of a sealing plug length *Le* and a support body trailing end length *Ls.* The trailing end portion 26 of the rigid support body extends over the axial trailing end length Ls behind the elastic sealing plug 12.

The plug insert portion 24 of the support body 14 comprises a head portion 28 and annular recess 30 interconnecting the head portion to the trailing end portion 26. The elastic sealing plug 12 comprises a complementary inner annular ring portion that fits within the annular recess 30, and the head portion 28 fits within the inner cavity 20 such that the elastic sealing plug 12 is securely fixed to the rigid support body 14. The head portion 28 further provides radial support to the tube portion 18 of the elastic sealing plug to prevent the tube portion 18 from collapsing radially and to ensure that at least one of the sealing ring lips 22 is compressed against the container inner wall.

The tube portion 18 comprises at least two sealing lips 22, whereby at least one sealing lip is axially aligned with and surrounds the annular recess 30 or the head portion 28. In an embodiment, at least one of the sealing lips 22 is axially aligned with and surrounds the head portion 28.

Preferably, the tube portion comprises at least three sealing ring lips 22, whereby preferably at least two of the sealing ring lips are axially aligned with and surround the head portion 28 and/or annular recess 30. In an embodiment, at least one of the sealing lips 22 is axially aligned with and surrounds the annular recess 30 and at least one of the sealing lips 22 is axially aligned with and surrounds the head portion 28.

In an embodiment, at least one sealing ring lip 22a extends around the front wall portion 16 of the elastic sealing plug 12, axially offset from an end of the head portion 28 of the rigid support body 14.

In order to ensure an advantageous relationship between the compressibility of the elastic sealing plug 12 and stable support and anchoring of the rigid support body 14 to the elastic sealing plug 12, the head portion 28 preferably has a diameter *Ds* that is at least 60%, preferably at least 65% of the diameter *D* of the plunger.

The trailing end portion 26 of the rigid support body comprises one or more guide rims 32 having radial guide surfaces 44 that slidably engage the container wall 8 over the length *Ls* of the support body trailing end portion. In an embodiment where there is more than one guide rim, the guide rims are separated by an annular recess 34. In an embodiment with only one guide rim, the guide rim may either extend axially over the entire trailing end length Ls, or only a portion thereof, positioned at the rear end of the plunger and separated from the elastic sealing plug by an annular recess.

In an embodiment, as illustrated, at least one guide rim 32 is arranged at an end of the trailing end portion 26 and at least the second guide rim 32 is positioned adjacent and in contact with a rear end of the elastic sealing plug 12.

The one or more guide rims 32 prevent tilting of the plunger 6 within the container as the plunger is drawn in by the negative pressure when liquid is pumped out by the suction pump 2. As negative pressure is applied within the container, the front wall portion 16 of the sealing plug is elastically deformed in an axial direction A by the traction effect of the negative pressure. Traction on the front wall portion 16 may slightly release compression pressure of a sealing ring lip 22a at a front end of the elastic sealing plug 12 thus assisting in reducing the stick-slip effect and facilitating sliding of the plunger under the effect of the negative pressure within the container. Also, the negative pressure provides some traction on the elastic sealing plug 6 that causes a slight axial extension (stretching) of the sealing plug that may assist in reducing the stick-slip effect compared to an elastic plunger that is advanced by compression by a piston rod.

In an advantageous embodiment, the front wall portion 16 of the elastic sealing plug has a thickness *Lt* that is in a range of 10% to 30% of the outer diameter *D* of the plunger to provide an optimal balance between the firmness and suppleness of the front sealing lip 22a in radial compression and axial traction.

In a variant, the elastic sealing plug 12 and rigid body 14 are manufactured as separate components and assembled together by inserting the head portion 28 into the cavity of the sealing plug. In another variant, the elastic sealing plug 12 is molded, for instance injection molded, around the head portion of the rigid body 14. In a variant, the elastic sealing plug 12 and rigid body 14 may be manufactured together in a multi-component injection moulding process.

A central cavity 36 extending axially into an end of the rigid body 14 may be provided to receive an assembly rod or other tool assisting assembly of the rigid body to the elastic sealing plug 12. The cavity may also serve to lodge a sensor element such as a magnet or conductive piece that may be detected by a position sensor on or outside the container 4.

The rigid support body including the head portion 28 mounted within the sealing plug and the trailing end portion 26 guiding the trailing end of the plunger within the container serve to prevent the elastic sealing plug from rotating into a cocked position that would potentially increase friction and block the sealing plug within the container, or alternatively cause a leak of the sealing rings thus allowing air to enter into the container. The magnitude of the plunger length *L* is preferably at least 0.8 times the value of the plunger outer diameter *D,* more preferably at least 1 times the value of the outer diameter *D* of the plunger.

In an embodiment, the support body trailing end length *Ls* is preferably at least 30% of the overall plunger length *L,* for instance in a range of 40-60% the overall plunger length *L.*

In an embodiment, the support body trailing end length *Ls* is preferably at least 30% of the overall plunger length *L,* for instance in a range of 40-60% the overall plunger length *L.*

Use of a conventional elastomeric plunger with a suction pump system may lead to cocking of the plunger and is thus unreliable and potentially unsafe. The plunger according to the invention with the rigid support body comprising a head portion mounted within the elastic sealing plug and the trailing end portion serving to guide the plunger and also provide to the certain length of rigid material that prevent cocking of the plunger allows the provision of a medical device with a rigid container which is compact and economical to produce, yet safe and reliable to use.

### List of features illustrated

*Drug delivery device 1*
   *Suction pump 2*
   ***Container 4***
      *Container wall 8*
      *Outlet 10*
   ***Plunger 6***
      ***Elastic sealing plug 12***
         *Front wall portion 16*
         *Tube portion 18*
            *Sealing ring lips 22, 22a*
         *Inner cavity 20*
      ***Rigid support body** 14*
         ***Plug insert portion 24***
            *Head portion 28*
               *Locking shoulder 38*
               *Leading end chamfer 40*
            *Annular recess 30*
               *Locking shoulder 42*
         ***Trailing end portion 26***
            *Guide rim(s) 32*
               *Radial guide surface(s) 44*
            *Annular recess 34*
            *Inner cavity 36*
*Plunger length L*
*Plunger diameter D*
*Plug insert head diameter Ds*
*Sealing plug length Le*
*Support body trailing Length Ls*
*Sealing plug front portion thickness Lt*

## Claims

1. Drug delivery device comprising a suction pump (2) and a liquid drug container (4), the container comprising a tubular container wall (8) extending from an outlet (10) fluidly connected to the suction pump (2) and a rear open end within which a plunger (6) is inserted, the plunger comprising an elastic sealing plug (12) with sealing ring lips (22) compressed against an inner surface of the tubular container wall hermetically sealing a liquid drug within the cartridge, **characterized in that** the plunger further comprises a rigid support body (14), the rigid support body comprising a plug insert portion (24) comprising a head portion (28) mounted within an inner cavity (20) of the elastic sealing plug (12) and a trailing end portion (26) extending rearwardly from the head portion (28) and comprising at least one guide rim (32) having a radial guide surface (44) slidably engaging the container wall, whereby said at least one guide rim (32) extends up to a distance (*L*) from a front wall portion (16) of the elastic sealing plug (12) of at least 80% of an overall outer diameter (*D*) of the plunger.

2. The drug delivery device according to the preceding claim wherein the trailing end portion (26) of the rigid support body (14) comprises at least two guide rims separated by an annular recess (34).

3. The device according to any of the preceding claim wherein said at least one guide rim (32) is arranged at an end of the trailing end portion (26) and at least the second guide rim (32) is positioned adjacent and in contact with a rear end of the elastic sealing plug (12).

4. The device according to any of the preceding claim wherein the head portion (28) of the rigid support body (14) comprises an outer diameter (*Ds*) that is at least 60% of an outer diameter (*D*) of the plunger.

5. The device according to any of the preceding claim wherein the plug insert portion comprises an annular recess (30) interconnecting the head portion (28) to the trailing end portion (26), and the elastic sealing plug (12) comprising an inner annular ring portion complementary to the annular recess and mounted in the annular recess of the plug insert portion for fixedly coupling the elastic sealing plug to the rigid support body (14).

6. The device according to any of the preceding claim wherein the elastic sealing plug comprises at least two sealing ring lips (22), at least one of the sealing ring lips being positioned around and axially aligned with the head portion (22) of the rigid support body (14).

7. The device according to the preceding claim wherein the elastic sealing ring comprises at least three sealing ring lips (22), at least two of the sealing ring lips axially aligned with and extending around the plug insert portion (24).

8. The device according to the preceding claim wherein the elastic sealing plug (12) comprises at least one sealing ring lip (22) extending around a front wall portion (16) of the elastic sealing plug (12) axially offset from an end of the head portion (28) of the rigid support body (14).

9. The device according to any preceding claim wherein a front wall portion (16) of the elastic sealing plug has a thickness (*Lt*) in a range of 10% to 30% of an outer diameter (*D*) of the plunger.

10. The device according to any preceding claim wherein the rigid support body trailing end length (*Ls*) is at least 30% of the overall plunger length (*L*).

11. The device according to any preceding claim wherein the rigid support body trailing end length (*Ls*) is in a range of 40-60% of the overall plunger length (*L*).
